# EUROPEAN PATENT SPECIFICATION

(11) **EP 1 626 949 B1**
(45) Date of publication and mention of the grant of the patent: **21.03.2007**
(21) Application number: 04754526.4
(22) Date of filing: 07.06.2004
(51) Int. Cl.: C07C 67/303, C07C 51/36, B01J 31/18

(54) **SUPPORTED METAL-PHOSPHINE-AMINOPHOSPHINE CATALYSTS**
GETRÄGERTE METALL-PHOSPHIN-AMINOPHOSPHIN-KATALYSATOREN
CATALYSEURS SUPPORTES DE METAL-PHOSPHINE-AMINOPHOSPHINE

(30) Priority: 05.06.2003 US 476059 P
(43) Date of publication of application: 22.02.2006
(73) Proprietor: EASTMAN CHEMICAL COMPANY, Kingsport, TN 37660 (US); Seton Hall University, South Orange, NJ 07079 (US)
(72) Inventor: AUGUSTINE, Robert, Leo, Livingston, NJ 7039 (US); TANIELYAN, Setrak, Kavork, Maplewood, NJ 7040 (US); REYES, Clementine, Lawrenceville, NJ 8648 (US); BOAZ, Neil, Warren, Kingsport, TN 37660 (US)
(74) Representative: Wibbelmann, Jobst
(86) International application number: PCT/US2004/017935
(87) International publication number: WO 2004/108651

(56) References cited:
- US-A- 6 025 295
- US-A- 6 136 746
- US-A1- 2002 065 417
- BOAZ, NEIL W. ET AL: "Phosphinoferrocenylaminophosphines as Novel and Practical Ligands for Asymmetric Catalysis" ORGANIC LETTERS , 4(14), 2421-2424 CODEN: ORLEF7; ISSN: 1523-7060, 2002, XP002297176
- CARPENTIER, JEAN-FRANCOIS ET AL: "Catalytic asymmetric hydrogenation of activated keto compounds by some homogeneous and silica- supported di(.mu.-carboxylato)bis( aminophosphinephosphinite )dirhodium complexes" TETRAHEDRON: ASYMMETRY , 6(1), 39-42 CODEN: TASYE3; ISSN: 0957-4166, 1995, XP002297177

## Description

### Field of the Invention

The present invention relates to a re-usable and highly stable, supported complex which exhibits high reactivity, stability, and selectivity for asymmetric catalytic reactions. More specifically, the present invention relates to a supported metal complex of a substantially enantiomerically pure phosphine-aminophosphine ligand which comprises a support, an anchoring agent, and a metal complex of a substantially enantiomerically pure phosphine-aminophosphine ligand. This invention also pertains to novel methods for the preparation of the supported catalyst complex and their use for asymmetric catalytic reactions.

### Background of the Invention

Asymmetric catalysis is the most efficient method for generating products having high enantiomeric purity, as the asymmetry of the catalyst is multiplied many times over in the generation of the chiral product. These chiral products have found numerous applications as building blocks for single enantiomer pharmaceuticals as well as in some agrochemicals. The asymmetric catalysts employed can be enzymatic or synthetic in nature. The latter types of catalyst have much greater promise than the former due to much greater latitude of applicable reaction types. Synthetic asymmetric catalysts are usually composed of a metal reaction center surrounded by an organic ligand. The ligand usually is generated in high enantiomeric purity, and is the agent inducing the asymmetry. These ligands are, in general, difficult to make and therefore expensive. In addition, these ligand generally cannot be re-used due to, for example, difficulty in recovery and instability upon re-isolation.

The main strategy used to affix a homogeneous catalyst to a support (e.g., to heterogenize it) is to modify the ligand such that it can be incorporated into a polymer matrix. Unfortunately, such methods generally result in materials that exhibit significant loss of both activity and selectivity as compared to the original, soluble catalyst. Further, the activity of the heterogenized species often degrades rapidly upon attempted re-use. The supported catalysts described by Tanielyan et al, in United States Patents 6,005,148, 6,025,295 and 6,136,746 utilize a novel mode of immobilization that does not involve covalent modification of the chiral ligand. These supported species generally exhibit selectivity comparable to the homogeneous counterparts and maintain their catalytic activity over multiple re-uses.

The preparation and use of metal complexes of substantially enantiomerically pure phosphine-aminophosphine ligands for asymmetric catalysis has been described by Boaz et al. in published United States Patent application 20020065417. There has been no report of heterogenization of metal complexes of phosphine-aminophosphine ligands.

### Brief Description of the Figures

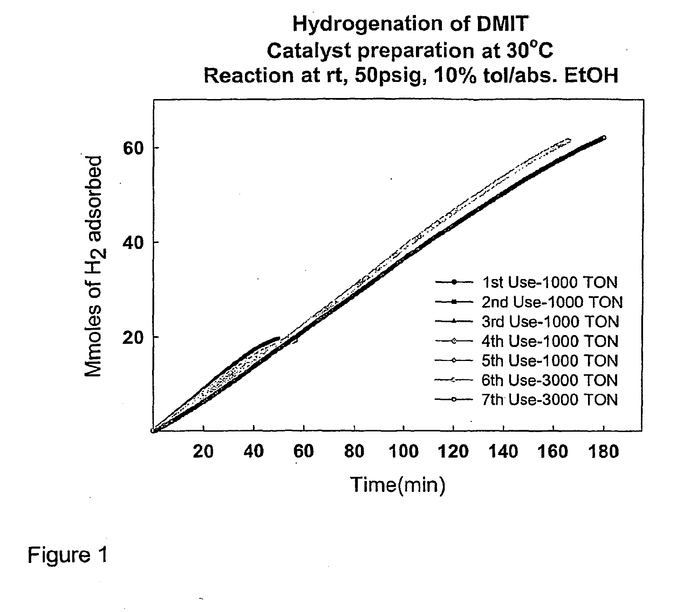

Figure 1 depicts the rate of hydrogenation of dimethylitaconate using a supported metal-phosphine-aminophosphine catalyst prepared from a support, an anchoring agent, and a metal complex of a substantially enantiomerically pure phosphine-aminophosphine. This figure shows similar catalyst activity through seven uses of the same supported catalyst. Figure 1 reflects the experimental work in Example 3.

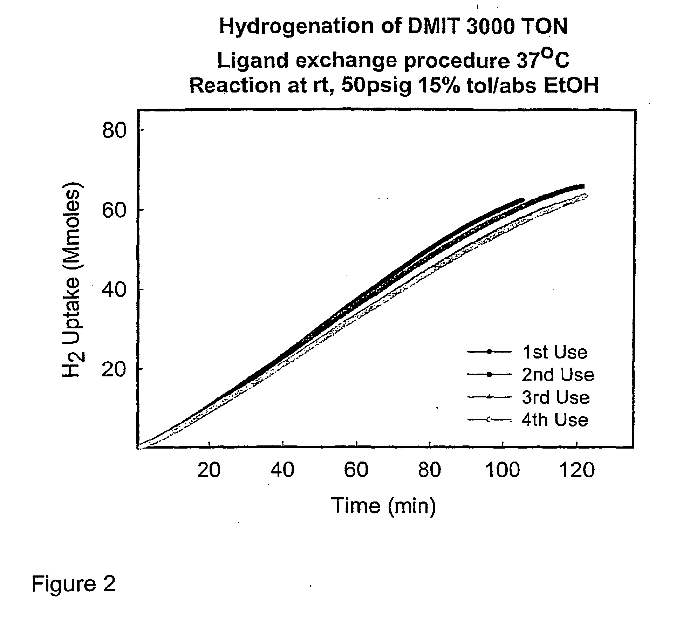

Figure 2 depicts the rate of hydrogenation of dimethyl itaconate using a supported metal-phosphine-aminophosphine prepared from a supported catalyst precursor and a substantially enantiomerically pure phosphine-aminophosphine. This figure shows similar catalyst activity through four uses of the same supported catalyst. Figure 2 reflects the experimental work in Example 4.

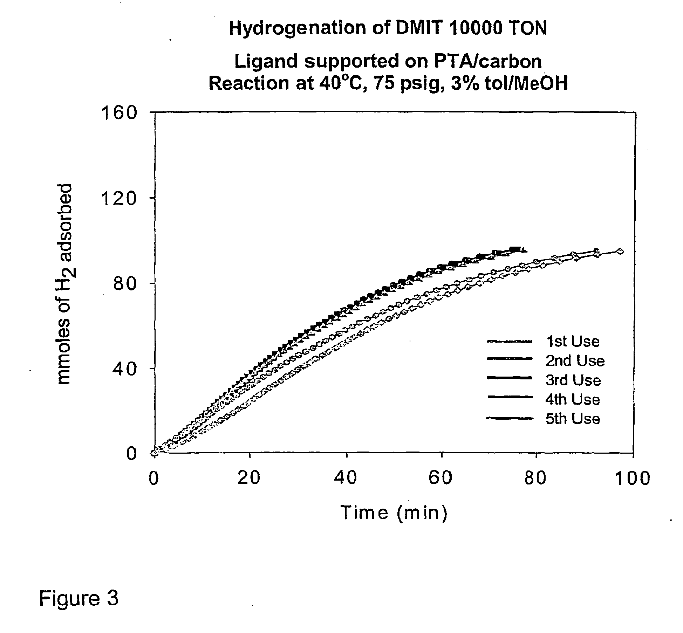

Figure 3 depicts the rate of hydrogenation of dimethyl itaconate using a supported metal-phosphine-aminophosphine prepared from a supported catalyst precursor and a substantially enantiomerically pure phosphine-aminophosphine (e.g., hydrogenation of dimethyl itaconate using C/PTA/Rh-(R)-1 at S:C 10,000). This figure shows similar catalyst activity through five uses of the same supported catalyst. Figure 3 reflects the experimental work in Example 5.

### Brief Summary of the Invention

The novel supported catalysts of our invention comprise a support, an anchoring agent, and a metal complex of a substantially enantiomerically pure phosphine-aminophosphine ligand. These species are useful for asymmetric catalytic reactions, particularly.for asymmetric hydrogenation. These supported catalysts have good activity, afford high enantioselectivity, and, surprisingly, can be re-used with minimal loss of catalytic activity.

### Detailed Description

As stated above, the present invention comprises a supported catalyst comprising a support, an anchoring agent, and a metal complex of a substantially enantiomerically pure phosphine-aminophosphine ligand. The term "substantially enantiomerically pure" is meant to indicate that the ligand is characterized by a >90% enantiomeric excess [ee] of the indicated enantiomer.

The support for use in the present invention comprises an insoluble, particulate amorphous or crystalline material having a sufficient surface area to facilitate uniform distribution of the anchoring agent. Suitable supports include those that are readily and generally commercially available or may be prepared by techniques known to those of skill in the art. Examples of appropriate support materials include, but are not limited to: metal oxides such as alumina, silica, titania, zirconia, lanthana, zeolites, and clays, as well as carbons, resins, polymers, and the like.

The support may be used as is (e.g., as purchased or prepared), or it may be treated prior to further use to remove unwanted species that may adversely effect the activity of the overall catalyst complex. For example, the support may be calcined either in air or in an inert atmosphere prior to use. Similarly, the support may be treated with a modifier to enhance adhesion of the anchoring agent. Suitable modifiers that may be used include, but are not limited to: metal alkoxides such as titanium alkoxide, aluminum alkoxide, silane alkoxide, vanadium alkoxide and the like; polyisocyanates, hydroxyepoxides, cyanoepoxides, and other functionalized organic materials.

The anchoring agent or anchor is a heteropoly acid or anion. The term "heteropoly acid" (or anion) as used herein denotes any polyprotic mixed oxide that is generally comprised of a central ion or ions bonded to an appropriate number of oxygen atoms and surrounded by a near spherical shell of octahedral oxometal species joined together by shared oxygen atoms. The central atom or "heteroatom" is typically a cation having a +3 to +5 oxidation state such as P(+5), As(+5), Si(+4), or Mn(+4). The metal species associated with the octahedral are usually Mo, W, or V. The octahedral in the shell of the heteropoly acid can be of uniform composition or contain different metal species. Suitable heteropoly acids include, but are not limited to: phosphotungstic acid (PTA), phosphomolybdic acid (PMA), silicotungstic acid (STA) and the like; Dawson species; Waugh species; Anderson species; Silverton species; their lacunar and other crystalline or non-crystalline forms; and anions of the preceding. When a heteropoly anion is used, the counterions may be any species that readily affords the appropriate heteropoly anion. Suitable counterions (or cations) include, but are not limited to: alkali, alkaline earth, or quaternary ammonium ions.

The metal complex of the substantially enantiomerically pure phosphine-amino phosphine ligand is a complex of one or more Group IIIB, IVB, VB, VIB, VIIB, VIII, IB, or IIB metals, and a substantially enantiomerically pure bis-phosphine compound comprising a substantially enantiomerically pure chiral backbone linking two phosphine residues wherein one of the phosphine residues has three phosphorus-carbon bonds and the other phosphine residue has two phophorus-carbon bonds and one phosphorus-nitrogen bond wherein the nitrogen is part of the chiral backbone. Examples of preferred metals for use with the subject ligand include Group VIII metals such as rhodium, iridium or ruthenium.

Examples of the substantially enantiomerically pure, compounds (i.e., the subject-ligand) include phosphinometallocenyl-aminophosphines having the general formulas **1** or **2** (the enantiomer of **1**): 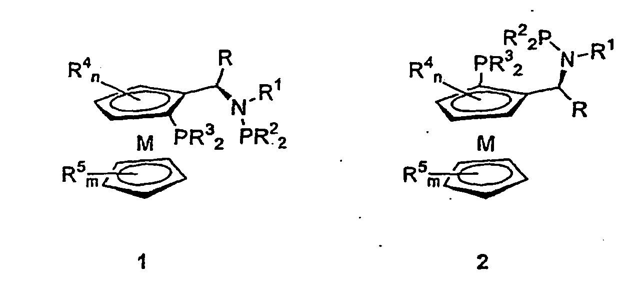 wherein
R is selected from substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
R¹, R², R³, R⁴, and R⁵ are independently selected from hydrogen, substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
n is 0 to 3;
m is 0 to 5; and
M is selected from the metals of Groups IVB, VB, VIB, VIIB and VIII.

The alkyl groups which may be represented by each of R, R¹, R², R³, R⁴, and R⁵ may be straight- or branched-chain, aliphatic hydrocarbon radicals containing up to about 20 carbon atoms and may be substituted, for example, with one to three groups selected from C₁-C₆-alkoxy, cyano, C₂-C₆-alkoxycarbonyl, C₂-C₆-alkanoyloxy, hydroxy, aryl and halogen. The terms "C₁-C₆alkoxy", "C₂-C₆-alkoxycarbonyl", and "C₂-C₆-alkanoyloxy" are used to denote radicals corresponding to the structures -OR⁶, -CO₂R⁶, and -OCOR⁶, respectively, wherein R⁶ is C₁-C₆-alkyl or substituted C₁-C₆-alkyl. The term "C₃-C₈-cycloalkyl" is used to denote a saturated, carbocyclic hydrocarbon radical having three to eight carbon atoms. The aryl groups which each of R, R¹ R², R³, R⁴, and R⁵ may represent may include phenyl, naphthyl, or anthracenyl and phenyl, naphthyl, or anthracenyl substituted with one to three substituents selected from C₁-C₆-alkyl, substituted C₁-C₆-alkyl, C₆-C₁₀ aryl, substituted C₆-C₁₀ aryl, C₁-C₆-alkoxy, halogen, carboxy, cyano, C₁-C₆-alkanoyloxy, C₁-C₆-alkylthio, C₁-C₆-alkylsulfonyl, trifluoromethyl, hydroxy, C₂-C₆-alkoxycarbonyl, C₂-C₆-alkanoylamino and -O-R⁷, S-R⁷,-SO₂-R⁷, -NHSO₂R⁷ and -NHCO₂R⁷, wherein R⁷ is phenyl, naphthyl, or phenyl or naphthyl substituted with one to three groups selected from C₁-C₆-alkyl, C₆-C₁₀ aryl, C₁-C₆-alkoxy and halogen.

The heteroaryl radicals include a 5- or 6- membered aromatic ring containing one to three heteroatoms selected from oxygen, sulfur and nitrogen. Examples of such heteroaryl groups are thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, pyridyl, pyrimidyl, benzoxazolyl, benzothiazolyl, benzimidazolyl, indolyl and the like. The heteroaryl radicals may be substituted, for example, with up to three groups such as C₁-C₆-alkyl, C₁-C₆-alkoxy, substituted C₁-C₆-alkyl, halogen, C₁-C₆-alkylthio, aryl, arylthio, aryloxy, C₂-C₆-alkoxycarbonyl and C₂-C₆-alkanoylamino. The heteroaryl radicals also may be substituted with a fused ring system, e.g., a benzo or naphtho residue, which may be unsubstituted or substituted, for example, with up to three of the groups set forth in the preceding sentence. The term "halogen" is used to include fluorine, chlorine, bromine, and iodine.

Examples of preferred ligands of the invention have formulas 1 or 2 wherein R is C₁ to C₆ alkyl; R¹ is hydrogen or C₁ to C₆ alkyl; R² is aryl (preferably phenyl), ethyl, isopropyl, or cyclohexyl; R³ is aryl, most preferably phenyl; R⁴ and R⁵ are hydrogen; and M is iron, ruthenium, or osmium, most preferably iron. The foregoing ligand-metal complexes may further comprise one or more achiral ligands which include, but are not limited to: species such as cyclopentadiene, carbon monoxide, cyclooctadiene, norbomadiene, and tertiary phosphines.

The present invention also provides for the preparation of the supported catalyst complexes described above. A method for preparing the supported catalysts may involve contacting a support with a heteropoly acid or anion anchoring agent under conditions effective to form a heteropoly acid or anion-containing support, and then contacting a metal complex of a substantially enantiomerically pure phosphine-aminophosphine with the heteropoly acid or anion-containing support under conditions effective to form a supported catalyst.

Further, the supported catalyst of the present invention may be prepared by contacting a previously prepared support and anchor combination with a metal complex of the substantially enantiomerically pure phosphine-aminophosphine ligand. The preparation of the anchor-support is as described in Tanielyan et al. in United States Patents 6,005,148, 6,025,295 and 6,136,746. Likewise, the preparation of the phosphine-aminophosphine ligand and its metal complex is as described in Boaz et al. in published United States Patent application 20020065417.

The contacting of the ligand-metal complex and the support-anchor combination typically occurs in a solvent and at a temperature from about - 25°C to about 250°C for a time period from about 1 min to about 50 hrs. Preferably this contacting takes place at temperatures between about 25°C and 50°C for a time period of between about 1 hr and about 24 hrs. Generally, the ligand-metal complex is employed at a concentration such that the metal complex to heteropolyacid or anion anchor material molar ratio is from about 0.1:1 to about 6:1; more preferably the ratio is from about 0.5:1 to about 1.5:1. The solvent employed is capable of dissolving the metal complexes. Preferred solvents include lower alcohols (C1-C5), ethers, esters, ketones, and aliphatic or aromatic hydrocarbons or mixtures thereof.

The resulting solid, supported catalyst of the invention may then be optionally recovered using techniques known to those skilled in the art, such as decantation, filtration, or centrifugation. The solid supported catalyst may be used as is, or it optionally may be washed with one of the aforementioned solvents prior to use to remove any non-supported material.

An alternative method of preparing the solid catalysts of the invention involves first contacting the anchoring agent with the ligand-metal complex of the substantially enantiomerically pure phosphine-aminophosphine to form a mixture, and then that mixture is contacted with a support material. The foregoing mixture may take the form of either a suspension or solution.

The contacting between the anchoring agent and the metal complex of the substantially enantiomerically pure phosphine-aminophosphine typically occurs in a solvent and at a temperature from about -25°C to about 250°C for a time period from about 1 min to about 50 hrs. Preferably this contacting takes place at temperatures between about 25°C and about 50°C for a time period of between about 1 hr and about 24 hrs. Generally; in the present invention, the metal complex is employed at a concentration such that the metal complex to heteropolyacid or anion molar ratio is from about 0.1:1 to about 6:1, more preferably from about 0.5:1 to about 1.5:1.

The resulting mixture containing the anchoring agent and the metal complex may be contacted with the support material as is, or, alternatively, the resulting solution or suspension may be dried and then slurried in a comparable solvent prior to contacting with one of the support materials set forth above. This contacting generally occurs in a solvent at a temperature of from about -25°C to about 250°C for a period of time from about 1 min to 50 hrs. Preferably, this contacting takes place at a temperature of about 25°C to about 50°C for a period of time from about 3 hrs to about 12 hrs. In accordance with this aspect of the present invention, the anchoring agent and metal complex solution or suspension is present in about 0.01% to about 150% by weight of the support employed in this contacting step. Solvents that may be used in either step of this embodiment include lower alcohols (C1-C5), ethers, esters, ketones, and aliphatic or aromatic hydrocarbons or mixtures thereof.

The solid supported catalyst of this invention may then be optionally recovered using techniques known to those skilled in the art, such as decantation, filtration, or centrifugation. The solid supported catalyst may be used as is, or it optionally may be washed with one of the aforementioned solvents prior to use to remove any non-supported material.

In a further embodiment of the present invention, a supported catalyst complex is prepared by contacting a supported catalyst precursor with a substantially enantiomerically pure phosphine-aminophosphine ligand. The preparation of a supported, metal-containing, catalyst precursor is described by Tanielyan et al. in the United States Patents identified above. Once prepared, the supported catalyst precursor is then contacted with a substantially enantiomerically pure phosphine-aminophosphine ligand, described hereinabove, to form the supported metal complex.

The supported catalyst precursor for use herein comprises a support, an anchoring agent, and a metal salt or complex from which a catalytically active entity may be prepared. Examples of catalyst precursor materials include rhodium cyclooctadiene dimer, bis(cyclooctadiene)rhodium tetrafluoroborate, bis(cyclooctadiene)rhodium trifluoromethanesulfonate, bis(norbomadiene)rhodium tetrafluoroborate, ruthenium cyclooctadiene dimer, allyl palladium chloride dimer, and rhodium chloride.

The concentration of the substantially enantiomerically pure phosphine-aminophosphine ligand that is added to the supported catalyst precursor is typically about 1 to about 6 mmol per mmol of supported catalyst precursor material. The treatment of the supported catalyst precursor with the substantially enantiomerically pure phosphine-aminophosphine ligand typically occurs in a solvent at temperatures of from about -25°C to about 250°C, and preferably about 25° to about 50°C, for a period of time from about 1 min to about 50 hrs. Preferred solvents for this embodiment include lower alcohols (C1-C5), ethers, esters, ketones, and aliphatic or aromatic hydrocarbons or mixtures thereof.

The solid supported catalyst of this invention may then be optionally recovered using techniques known to those skilled in the art, such as decantation, filtration, or centrifugation. The solid supported catalyst may be used as is, or it optionally may be washed with one of the aforementioned solvents prior to use to remove any non-supported material.

These supported complexes of the present invention may be used to perform asymmetric catalytic reactions. Examples of such reactions include, but are not limited to: asymmetric hydrogenations, asymmetric reductions, asymmetric hydroborations, asymmetric olefin isomerizations, asymmetric hydrosilations; asymmetric allylations, asymmetric hydroformylations, and asymmetric organometallic additions. A preferred embodiment of the present invention is the use of the supported complexes for asymmetric hydrogenation reactions, an example of which is the catalytic asymmetric hydrogenation of prochiral substituted α,β-unsaturated acids or esters or other prochiral substrates to provide the saturated, chiral counterpart. The hydrogenation conditions employed in the present invention are those that are typically employed by those of skill in the art for carrying out such a reaction.

The amount of supported catalyst complex employed may vary between 0.000005 and 0.5 equivalents based on the reactant compound, with more complex usually providing faster reaction rates. The atmosphere for the reaction is hydrogen, but may also contain other gases or materials that are inert to the reaction conditions. The reaction can be run at atmospheric pressure or at elevated pressure, generally from about 0.5 to about 200 bars gauge (barg). The reaction is run at a temperature which affords a reasonable rate of conversion, which can be as low as -50°C but is usually between ambient temperature and the boiling point (or apparent boiling point at elevated pressure) of the lowest boiling component of the reaction mixture. The reaction may be run in the presence of a solvent chosen from aliphatic hydrocarbons such as hexane, heptane, octane and the like, aromatic hydrocarbons such as toluene, xylenes, and the like, cyclic or acyclic ethers such as tert-butyl methyl ether, diisopropyl ether, tetrahydrofuran and the like, lower alcohols such as methanol, ethanol, n-propanol, isopropanol, n-butanol and the like, halogenated aliphatic or aromatic hydrocarbons such as dichloromethane, tetrachloroethylene, chloroform, chlorobenzene and the like, esters such as methyl acetate, ethyl acetate, methyl propionate, isopropyl acetate and the like, dialkyl ketones such as acetone, 2-butanone, 3-pentanone, methyl isopropyl ketone, methyl isobutyl ketone and the like, or polar aprotic solvents such as dimethylformamide, dimethyl sulfoxide and the like or mixtures thereof. The products of such reaction are generally obtained in high enantiomeric excess (>85% ee).

As stated previously, the supported catalysts described herein may be re-used multiple times without substantial loss of catalytic activity or enantioselectivity. The ability to re-use the supported catalysts of this invention is surprising and unexpected because the skilled artisan would expect a phosphine-aminophosphine catalyst to degrade or deactivate due to cleavage of the nitrogen-phosphorus bond of the aminophosphine over continued uses even if one were able to affix it to a support. This is particularly true when such phosphine-aminophosphine catalysts are exposed to alcoholic solvents. Clearly, cleavage of the nitrogen-phosphorus bond would result in loss of catalytic activity and enantioselectivity.

### Examples

The novel compounds and processes provided by the present invention are further illustrated by the following examples.

### Example 1

A reactor containing Al₂O₃/PTA support (0.4221g) was evacuated and refilled with argon several times before adding 15 mL of degassed absolute ethanol via cannula. After this addition, the cannula was removed and the reactor evacuated for about 10 minutes or until no bubbling was observed. During this time, bis(norbomadiene)rhodium tetrafluoroborate (0.0075 g; 20 micromoles) and (R)-N-diphenylphosphino-1-[(S)-2-(diphenylphosphino)-ferrocenyl]ethylamine (1, R = R¹ = CH₃, R² = R³ = Ph, n = m = 0, M = Fe)(0.0122 g; 20 micromoles) were weighed and transferred to a 10 mL volumetric flask. After evacuating the contents of the flask for about 5 minutes, the contents were diluted to the mark with absolute ethanol and sonicated for 25 minutes. The contents of this flask were then added to the reactor via cannula at a slow rate (~10 minutes). Upon complete addition and removal of the cannula, the reactor was evacuated, refilled with argon at least 3 times and then placed in a water bath (37°C) for overnight stirring (~550 rpm). The next day, the support was washed 3-4 times with absolute ethanol and then dried under vacuum.

### Example 2

A reactor containing Al₂O₃/PTA support (0.4221 g) was evacuated and refilled with argon several times before adding 15 mL of degassed absolute ethanol via cannula. After this addition, the cannula was removed and the reactor evacuated for about 10 minutes or until no bubbling was observed. During this time, bis(cyclooctadiene)rhodium tetrafluoroborate (0.0081 g; 20 micromoles) was weighed and transferred to a 10 mL volumetric flask. After evacuating the contents of the flask for about 5 minutes, the contents were diluted to the mark with absolute ethanol. The contents of this flask were then added to the reactor via cannula at a slow rate (~10 minutes). Upon complete addition and removal of the cannula, the reactor was evacuated, refilled with argon at least 3 times and then placed in a water bath (37°C) for overnight stirring (~550 rpm). The next day, the support was washed 2 times with absolute ethanol. At this point, the support can be dried for further use or re-suspended in 15 mL of absolute ethanol. A solution containing 20 micromoles of (R)-N-diphenylphosphino-1-[(S)-2-(diphenylphosphino)-ferrocenyl]ethylamine (1, R = R¹ = CH₃, R² = R³ = Ph, n = m = 0, M = Fe) (0.0122g) was added dropwise to the support and the contents stirred overnight at a temperature of 37°C. The next day, the support was washed 3-4 times with absolute ethanol and then dried under vacuum.

### Example 3

The supported complex described in Example 1 was connected to a hydrogen line and the entire system was evacuated 8 times before exposing the support to hydrogen. A color change was observed as soon as the support was exposed to hydrogen (from orange to red-orange). At this point 15 mL of 15%toluene/abs. ethanol was added via syringe followed by the desired amount of dimethyl itaconate (8.5mL for a 3000 turnover number reaction). The hydrogenation was performed at ambient temperature under 50 psig of hydrogen and followed by hydrogen uptake. Upon completion of the reaction, the supernatant was removed and the reactor was charged with a second batch of solvent and substrate as described above. The supported complex was used for a total of seven runs. The rate data from these runs is graphed in Figure 1 and the turnover frequencies and enantiomeric excesses of the product is shown in Table 1.

**Table 1. TON is total supported catalyst turnover number. TOF is the catalyst turnover frequency per hour at the initial stages of the reaction.**

| Use No. | TON | TOF (Hr⁻¹) | % e.e. |
|---|---|---|---|
| 1 | 1000 | 1140 | 91% |
| 2 | 1000 | 1260 | 92% |
| 3 | 1000 | 1200 | 92% |
| 4 | 1000 | 1140 | 92% |
| 5 | 1000 | 1080 | 92% |
| 6 | 3000 | 1050 | 92% |
| 7 | 3000 | 1050 | 92% |

### Example 4

The supported complex described in Example 2 was connected to a hydrogen line and the entire system was evacuated, 8 times before exposing the support to hydrogen. A color change was observed as soon as the support was exposed to hydrogen (from orange to red-orange). At this point 15 mL of 15%toluene/abs. ethanol were added via syringe followed by the desired amount of dimethyl itaconate (8.5mL for a 3000 turnover number reaction). The hydrogenation was performed at ambient temperature under 50 psig of hydrogen and followed by hydrogen uptake. Upon completion of the reaction, the supernatant was removed and the reactor was charged with a second batch of solvent and substrate as described above. The supported complex was used for a total of four runs. The rate data from these runs is graphed in Figure 2 and the turnvover frequencies and enantiomeric excesses of the product is shown in Table 2.

**Table 2. TON is total supported catalyst turnover number. TOF is the catalyst turnover frequency per hour at the initial stages of the reaction.**

| Use No. | TON | TOF (Hr⁻¹) | % e.e. |
|---|---|---|---|
| 1 | 3000 | 1920 | 90.6% |
| 2 | 3000 | 1890 | 91.3% |
| 3 | 3000 | 1770 | 90.8% |
| 4 | 3000 | 1710 | 91.6% |

### Example 5

Phosphotungstic acid on carbon (0.164 g; contains 10 µmol of phosphotungstic acid) and 1,5-cyclooctadienerhodium-(R)-N-diphenylphosphino-1-[(S)-2-(diphenylphosphino)-ferrocenyl]ethylamine (10 µmol) were combined in 15 mL of 5% toluene in methanol under argon. The mixture was stirred overnight to afford a suspension of the supported catalyst The supernatant was removed via cannula, and the solid was washed until the washings were colorless. To this was added 40 mL of argon-degassed 3% toluene in methanol. The suspension was stirred and freshly purified dimethyl itaconate (14.1 mL; 100 mmol; S:C 10,000) was added. The reaction mixture was heated to 40°C and the argon atmosphere was replaced with 75 psig hydrogen. The reaction mixture was stirred and followed by hydrogen uptake. Upon completion of the reaction, the supernatant was removed and the reactor was charged with a second batch of solvent and substrate as described above. The supported complex was used for a total of five runs. The rate data from these runs is graphed in Figure 3 and the tumvover frequencies and enantiomeric excesses of the product is shown in Table 3.

**Table 3. TON is total supported catalyst turnover number. TOF is the catalyst turnover frequency per hour at the initial stages of the reaction.**

| Use No. | TON | TOF (Hr⁻¹) | % e.e. |
|---|---|---|---|
| 1 | 10000 | 10,400 | 92% |
| 2 | 10000 | 10,500 | 92% |
| 3 | 10000 | 10,500 | 92% |
| 4 | 10000 | 10,600 | 91% |
| 5 | 10000 | 9,200 | 92% |

### Example 6

Phosphotungstic acid on carbon (0.082 g; contains 5 µmol of phosphotungstic acid) and 1,5-cyclooctadienerhodium-(R)-N-diphenylphosphino-1-[(S)-2-(diphenylphosphino)-ferrocenyl]ethylamine (5 µmol) were combined in 15 mL of 5% toluene in methanol under argon. The mixture was stirred overnight to afford a suspension of the supported catalyst. The supernatant was removed via cannula, and the solid was washed until the washings were colorless. To this was added 120 mL of argon-degassed methanol. The suspension was stirred and freshly purified dimethyl itaconate (100 mL; 708 mmol; S:C 141,600) was added. The reaction mixture was heated to 50°C and the argon atmosphere was replaced with 75 psig hydrogen. The reaction mixture was stirred for about 15 hours to completely consume the dimethyl itaconate, affording dimethyl (R)-2-methylsuccinate with 88% ee and a catalytic activity of 12,500 turnovers per hour.

The invention has been described in detail with particular reference to preferred embodiments thereof, but it will be understood that variations and modifications can be effected within the spirit and scope of the invention.

## Claims

1. A supported catalyst comprising a support, an anchoring agent, and a metal complex of a substantially enantiomerically pure phosphine-aminophosphine ligand.

2. The supported catalyst of Claim 1 wherein said support is selected from the groups consisting of metal oxides, carbon, resins, and polymers.

3. The supported catalyst of Claim 2 wherein the metal oxide is selected from the group consisting of alumina, silica, titania, lanthana, zeolites, and clays.

4. The supported catalyst of Claim 2 wherein the support is treated by calcination or contacted with a modifier.

5. The supported catalyst of Claim 4 where the modifier is a metal alkoxide chosen from titanium alkoxide, aluminum alkoxide, silane alkoxide, or vanadium alkoxide.

6. The supported catalyst of Claim 1 wherein the anchoring agent is a heteropoly acid or anion selected from a Keggin type, a Dawson type, an Anderson type, or a Silverton type or mixtures thereof.

7. The supported catalyst of Claim 6 wherein the heteropoly acid is phosphotungstic acid, phosphomolybdic acid, silicotungstic acid, or the anions thereof.

8. The supported catalyst of Claim 1 wherein the metal complex comprises a metal from Groups IIIB, IVb, VB, VIB, VIIB, VIII, IB, or IIB.

9. The supported catalyst of Claim 8 wherein said metal is a Group VIII metal.

10. The supported catalyst of Claim 1 wherein the substantially enantiomerically pure phosphine-aminophosphine has formula **1**: 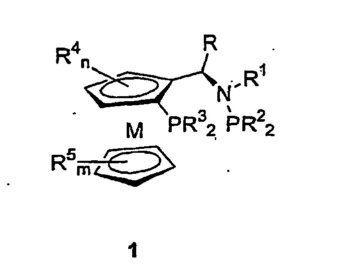 wherein
R is selected from substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
R¹, R², R³, R⁴, and R⁵ are independently selected from hydrogen, substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
n is 0 to 3;
m is 0 to 5; and
M is selected from the metals of Groups IVB, VB, VIB, VIIB and VIII.

11. The supported catalyst of Claim 1 wherein the substantially enantiomerically pure phosphine-aminophosphine has formula **2**: 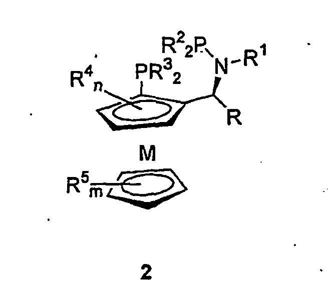 wherein
R is selected from substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
R¹, R², R³, R⁴, and R⁵ are independently selected from hydrogen, substituted and unsubstituted, branched- and straight-chain C₁-C₂₀ alkyl, substituted and unsubstituted C₃-C₈ cycloalkyl, substituted and unsubstituted C₆-C₂₀ carbocyclic aryl, and substituted and unsubstituted C₄-C₂₀ heteroaryl wherein the heteroatoms are selected from sulfur, nitrogen, and oxygen;
n is 0 to 3;
m is 0 to 5; and
M is selected from the metals of Groups IVB, VB, VIB, VIIB and VIII.

12. A method for preparing the supported catalyst of Claim 1 comprising:
(a) contacting a support with a heteropoly acid or anion anchoring agent under conditions effective to form a heteropoly acid or anion-containing support; and
(b) contacting a metal complex of a substantially enantiomerically pure phosphine-aminophosphine with the heteropoly acid or anion-containing support under conditions effective to form a supported catalyst.

13. The method of Claim 12 wherein step (a) occurs in a solvent at a temperature of from about -25°C to about 250°C for a period of time of from about 1 min to about 50 hrs.

14. The method of Claim 12 wherein step (b) occurs in a solvent at a temperature of from about -25°C to about 250°C for a period of time of from about 1 min to about 50 hrs.

15. The method of Claim 13 where the metal complex is contacted with the heteropoly acid or anion-containing support at a concentration to provide a molar ratio of metal complex to heteropoly acid or anion of from about 0.1:1 to about 6:1.

16. A method for the preparation of the supported catalyst of Claim 1 comprising:
(a) contacting a heteropoly acid or anion with a metal complex of a substantially enantiomerically pure phosphine-aminophosphine under conditions effective to form a mixture containing the heteropoly acid or anion and the metal complex; and
(b) contacting a support with the mixture formed in step (a) under conditions effective to form a supported catalyst.

17. The method of Claim 16 wherein step (a) occurs in a solvent at a temperature of from about-25°C to about 250°C for a period of time of from about 1 min to about 50 hrs.

18. The method of Claim 16 wherein the metal complex is contacted with the heteropoly acid or anion at a concentration to provide a molar ratio of metal complex to heteropoly acid or anion of from about 0.1:1 to about 6:1.

19. The method of Claim 16 wherein step (b) occurs in a solvent at a temperature of from about -25°C to about 250°C for a period of time of from about 1 min to about 50 hrs.

20. The method of Claim 16 where the heteropoly acid or anion and metal complex are present in a weight ratio of about 0.1:1 to about 20:1 as compared to the support employed in step (b).

21. A method of preparing the supported catalyst of Claim 1 comprising:
(a) contacting a support with a heteropoly acid or anion under conditions effective to form a heteropoly acid or anion-containing support;
(b) contacting said heteropoly acid or anion-containing support with a catalyst precursor material under conditions effective to form a supported catalyst precursor, and
(c) contacting said supported catalyst precursor with a substantially enantiomerically pure phosphine-aminophosphine ligand to provide a supported catalyst.

22. The method of Claim 21 wherein the support employed in step (a) is calcined or treated with a metal alkoxide prior to use.

23. The method of Claim 22 where the metal alkoxide is selected from the group consisting of titanium alkoxide, aluminum alkoxide, silane alkoxide, and vanadium alkoxide.

24. The method of Claim 21 wherein step (a) occurs in a solvent at a temperature of from about -25°C to about 250°C for a period of time of from about 1 min to about 50 hrs.

25. The method of Claim 21 where said catalyst precursor material is a metal salt or complex from which a catalytically active entity can be prepared.

26. The method of claim 25 where the metal salt or complex of said catalyst precursor is selected from the group consisting of rhodium cyclooctadiene dimer, bis(cyclooctadiene)rhodium tetrafluoroborate, bis(cycfooctadiene)rhodium trifluoromethanesulfonate, bis(norbomadiene)rhodium tetrafluoroborate, ruthenium cyclooctadiene dimer, allyl palladium chloride dimer, and rhodium chloride.

27. The method of Claim 21 wherein step (b) occurs in a solvent at a temperature of from about -25°C to about 250°C for a period of time of from about 1 min to about 50 hrs.

28. The method of Claim 21 wherein step (c) occurs in a solvent at a temperature of from about -25°C to about 250°C for a period of time of from about 1 min to about 50 hrs.

29. The method of Claim 21 wherein from about 1 mmol to about 6 mmol of ligand per mmol of supported catalyst precursor material is employed in step (c).

30. A process for the hydrogenation of an α,β-unsaturated acid or ester, which comprises contacting a prochiral α,β-unsaturated acid or ester with the supported catalyst of Claim 1 and hydrogen under conditions of hydrogenation temperature and pressure.

## Patentansprüche

1. Geträgerter Katalysator, umfassend einen Träger, einen Haftvermittler und einen Metallkomplex eines im Wesentlichen enantiomer reinen Phosphinaminophosphin-Liganden.

2. Geträgerter Katalysator nach Anspruch 1, bei dem der Träger ausgewählt ist aus der Gruppe bestehend aus Metalloxiden, Kohlenstoff, Harzen und Polymeren.

3. Geträgerter Katalysator nach Anspruch 2, bei dem das Metalloxid ausgewählt ist aus der Gruppe bestehend aus Aluminiumoxid, Siliciumdioxid, Titandioxid, Lanthanoxid, Zeolithen und Tonen.

4. Geträgerter Katalysator nach Anspruch 2, bei dem der Träger durch Kalzinierung behandelt oder mit einem Modifikationsmittel in Kontakt gebracht worden ist.

5. Geträgerter Katalysator nach Anspruch 4, bei dem das Modifikationsmittel ein Metallalkoholat ist, das aus Titanalkoholat, Aluminiumalkoholat, Silanalkoholat oder Vanadiumalkoholat ausgewählt ist.

6. Geträgerter Katalysator nach Anspruch 1, bei dem der Haftvermittler eine Heteropolysäure oder ein Anion ist, das ausgewählt ist aus einem Keggin-Typ, einem Dawson-Typ, einem Anderson-Typ oder einem Silverton-Typ oder aus deren Mischungen.

7. Geträgerter Katalysator nach Anspruch 6, bei dem es sich bei der Heteropolysäure um Phosphorwolframsäure, Phosphormolybdänsäure, Kieselwolframsäure oder deren Anionen handelt.

8. Geträgerter Katalysator nach Anspruch 1, bei dem der Metallkomplex ein Metall aus den Gruppen IIIB, IVb, VB, VIB, VIIB, VIII, IB oder IIB umfasst.

9. Geträgerter Katalysator nach Anspruch 8, bei dem das Metall ein Metall der Gruppe VIII ist.

10. Geträgerter Katalysator nach Anspruch 1, bei dem das im Wesentlichen enantiomer reine Phosphinaminophosphin die Formel **1** aufweist 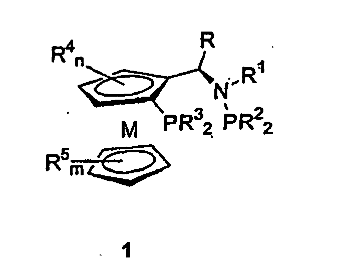 in der
R ausgewählt ist aus substituiertem und unsubstituiertem, verzweigt- und geradkettigem (C₁-C₂₀)-Alkyl, substituiertem und unsubstituiertem (C₃-C₈)-Cycloalkyl, substituiertem und unsubstituiertem carbocyclischem (C₆-C₂₀)-Aryl und substituiertem und unsubstituiertem (C₄-C₂₀)-Heteroaryl, worin die Heteroatome aus Schwefel, Stickstoff und Sauerstoff ausgewählt sind;
R¹, R², R³, R⁴ und R⁵ unabhängig ausgewählt sind aus Wasserstoff, substituiertem und unsubstituiertem, verzweigt- und geradkettigem (C₁-C₂₀)-Alkyl, substituiertem und unsubstituiertem (C₃-C₈)-Cycloalkyl, substituiertem und unsubstituiertem carbocyclischem (C₆-C₂₀)-Aryl und substituiertem und unsubstituiertem (C₄-C₂₀)-Heteroaryl, worin die Heteroatome aus Schwefel, Stickstoff und Sauerstoff ausgewählt sind;
n für 0 bis 3 steht;
m für 0 bis 5 steht; und
M aus den Metallen der Gruppen IVB, VB, VIB, VIIB und VIII ausgewählt ist.

11. Geträgerter Katalysator nach Anspruch 1, bei dem das im Wesentlichen enantiomer reine Phosphinaminophosphin die Formel **2** aufweist: 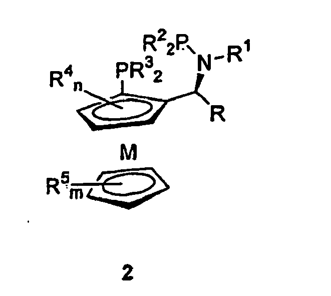 in der
R ausgewählt ist aus substituiertem und unsubstituiertem, verzweigt- und geradkettigem (C₁-C₂₀)-Alkyl, substituiertem und unsubstituiertem (C₃-C₈)-Cycloalkyl, substituiertem und unsubstituiertem carbocyclischem (C₆-C₂₀)-Aryl und substituiertem und unsubstituiertem (C₄-C₂₀)-Heteroaryl, worin die Heteroatome aus Schwefel, Stickstoff und Sauerstoff ausgewählt sind;
R¹, R², R³, R⁴ und R⁵ unabhängig ausgewählt sind aus Wasserstoff, substituiertem und unsubstituiertem, verzweigt- und geradkettigem (C₁-C₂₀)-Alkyl, substituiertem und unsubstituiertem (C₃-C₈)-Cycloalkyl, substituiertem und unsubstituiertem carbocyclischem (C₆-C₂₀)-Aryl und substituiertem und unsubstituiertem (C₄-C₂₀)-Heteroaryl, worin die Heteroatome aus Schwefel, Stickstoff und Sauerstoff ausgewählt sind;
n für 0 bis 3 steht;
m für 0 bis 5 steht; und
M aus den Metallen der Gruppen IVB, VB, VIB, VIIB und VIII ausgewählt sind.

12. Verfahren zur Herstellung des geträgerten Katalysators nach Anspruch 1, umfassend:
(a) In-Kontakt-Bringen eines Trägers mit einem Heteropolysäure- oder Anion-Haftvermittler unter Bedingungen, die wirksam sind, um einen Heteropolysäure- oder Anion-haltigen Träger zu bilden; und
(b) In-Kontakt-Bringen eines Metallkomplexes eines im Wesentlichen enantiomer reinen Phosphinaminophosphins mit dem Heteropolysäure- oder Anion-haltigen Träger unter Bedingungen, die wirksam sind, um einen geträgerten Katalysator zu bilden.

13. Verfahren nach Anspruch 12, bei dem der Schritt (a) in einem Lösungsmittel bei einer Temperatur von etwa -25°C bis etwa 250°C über eine Zeitspanne von etwa 1 min bis etwa 50 h stattfindet.

14. Verfahren nach Anspruch 12, bei dem der Schritt (b) in einem Lösungsmittel bei einer Temperatur von etwa -25°C bis etwa 250°C über eine Zeitspanne von etwa 1 min bis etwa 50 h stattfindet.

15. Verfahren nach Anspruch 13, bei dem der Metallkomplex mit dem Heteropolysäure- oder Anion-haltigen Träger bei einer solchen Konzentration in Kontakt gebracht wird, dass ein Molverhältnis von Metallkomplex zu Heteropolysäure oder Anion von etwa 0,1:1 bis etwa 6:1 bereitgestellt wird.

16. Verfahren zur Herstellung des geträgerten Katalysators nach Anspruch 1, umfassend:
(a) In-Kontakt-Bringen einer Heteropolysäure oder eines Anions mit einem Metallkomplex eines im Wesentlichen enantiomer reinen Phosphinaminophosphins unter Bedingungen, die wirksam sind, eine Mischung zu bilden, die die Heteropolysäure oder das Anion und den Metallkomplex enthält; und
(b) In-Kontakt-Bringen des Trägers mit der Mischung, die in Schritt (a) gebildet wurde, unter Bedingungen, die wirksam sind, um einen geträgerten Katalysator zu bilden.

17. Verfahren nach Anspruch 16, bei dem Schritt (a) in einem Lösungsmittel bei einer Temperatur von etwa -25°C bis etwa 250°C über eine Zeitspanne von etwa 1 min bis etwa 50 h stattfindet.

18. Verfahren nach Anspruch 16, bei dem der Metallkomplex mit der Heteropolysäure oder dem Anion bei einer solchen Konzentration in Kontakt gebracht wird, dass ein Molverhältnis von Metallkomplex zu Heteropolysäure oder Anion von etwa 0,1:1 bis etwa 6:1 bereitgestellt wird.

19. Verfahren nach Anspruch 16, bei dem Schritt (b) in einem Lösungsmittel bei einer Temperatur von etwa -25°C bis etwa 250°C über eine Zeitspanne von etwa 1 min bis etwa 50 h stattfindet.

20. Verfahren nach Anspruch 16, bei dem die Heteropolysäure oder das Anion und der Metallkomplex in einem Gewichtsverhältnis von etwa 0,1:1 bis etwa 20:1 im Vergleich zu dem in Schritt (b) verwendeten Träger vorliegen.

21. Verfahren zur Herstellung des geträgerten Katalysators nach Anspruch 1, umfassend:
(a) In-Kontakt-Bringen eines Trägers mit einer Heteropolysäure oder einem Anion unter Bedingungen, die wirksam sind, um einen Heteropolysäure- oder Anion-haltigen Träger zu bilden;
(b) In-Kontakt-Bringen des Heteropolysäure- oder Anion-haltigen Trägers mit einem Katalysatorvorstufen-Material unter Bedingungen, die wirksam sind, um eine geträgerte Katalysatorvorstufe zu bilden; und
(c) In-Kontakt-Bringen der geträgerten Katalysatorvorstufe mit einem im Wesentlichen enantiome reinen Phosphinaminophosphin-Liganden, um einen geträgerten Katalysator bereitzustellen.

22. Verfahren nach Anspruch 21, bei dem der in Schritt (a) verwendete Träger vor der Verwendung kalziniert oder mit einem Metallalkoholat behandelt wird.

23. Verfahren nach Anspruch 22, bei dem das Metallalkoholat ausgewählt ist aus der Gruppe bestehend aus Titanalkoholat, Aluminiumalkoholat, Silanalkoholat und Vanadiumalkoholat.

24. Verfahren nach Anspruch 21, bei dem Schritt (a) in einem Lösungsmittel bei einer Temperatur von etwa -25°C bis etwa 250°C über eine Zeitspanne von etwa 1 min bis etwa 50 h stattfindet.

25. Verfahren nach Anspruch 21, bei dem das Katalysatorvorstufen-Material ein Metallsalz oder -komplex ist, aus dem eine katalytisch aktive Einheit hergestellt werden kann.

26. Verfahren nach Anspruch 25, bei dem das Metallsalz oder der Metallkomplex der Katalysatorvorstufe ausgewählt ist aus der Gruppe bestehend aus Rhodiumcyclooctadien-Dimer, Bis(cyclooctadien)rhodiumtetrafluoroborat, Bis(cyclooctadien)rhodiumtrifiluormethansulfonat, Bis(norbornadien)rhodiumtetrafluoroborat, Rutheniumcyclooctadien-Dimer, Allylpalladiumchlorid-Dimer und Rhodiumchlorid.

27. Verfahren nach Anspruch 21, bei dem Schritt (b) in einem Lösungsmittel bei einer Temperatur von etwa -25°C bis etwa 250°C über eine Zeitspanne von etwa 1 min bis etwa 50 h stattfindet.

28. Verfahren nach Anspruch 21, bei dem Schritt (c) in einem Lösungsmittel bei einer Temperatur von etwa -25°C bis etwa 250°C über eine Zeitspanne von etwa 1 min bis etwa 50 h stattfindet.

29. Verfahren nach Anspruch 21, bei dem in Schritt (c) etwa 1 mMol bis etwa 6 mMol Ligand pro mMol geträgertes Katalysatorvorstufen-Material verwendet werden.

30. Verfahren zur Hydrierung einer α,β-ungesättigten Säure oder eines α,β-ungesättigten Esters, welches umfasst, dass man eine(n) prochirale(n) α,β-ungesättigte(n) Säure oder Ester mit dem geträgerten Katalysator nach Anspruch 1 und Wasserstoff unter Hydrierungstemperatur- und -druckbedingungen in Kontakt bringt.

## Revendications

1. Catalyseur supporté comprenant un support, un agent d'ancrage, et un complexe métallique d'un ligand de type phosphine - aminophosphine sensiblement énantiomériquement pure.

2. Catalyseur supporté selon la revendication 1, dans lequel ledit support est choisi dans le groupe comprenant les oxydes métalliques, le carbone, les résines, et les polymères.

3. Catalyseur supporté selon la revendication 2, dans lequel l'oxyde métallique est choisi dans le groupe comprenant l'alumine, la silice, le dioxyde de titane, le dioxyde de lanthane, les zéolites, et les argiles.

4. Catalyseur supporté selon la revendication 2, dans lequel le support est traité par calcination ou mis en contact avec un modificateur.

5. Catalyseur supporté selon la revendication 4, dans lequel le modificateur est un alcoxyde métallique choisi parmi l'alcoxyde de titane, l'alcoxyde d'aluminium, l'alcoxyde de silane, ou l'alcoxyde de vanadium.

6. Catalyseur supporté selon la revendication 1, dans lequel l'agent d'ancrage est un hétéropolyacide ou un hétéropolyanion choisi parmi un type Keggin, un type Dawson, un type Anderson, ou un type Silverton ou les mélanges de ceux-ci.

7. Catalyseur supporté selon la revendication 6, dans lequel l'hétéropolyacide est l'acide phosphotungstique, l'acide phosphomolybdique, l'acide silicotungstique, ou les anions de ceux-ci.

8. Catalyseur supporté selon la revendication 1, dans lequel le complexe métallique comprend un métal provenant des Groupes IIIB, IVB, VB, VIB, VIIB, VIII, IB, ou IIB.

9. Catalyseur supporté selon la revendication 8, dans lequel ledit métal est un métal du Groupe VIII.

10. Catalyseur supporté selon la revendication 1, dans lequel la phosphine - aminophosphine sensiblement énantiomériquement pure a la formule 1 : 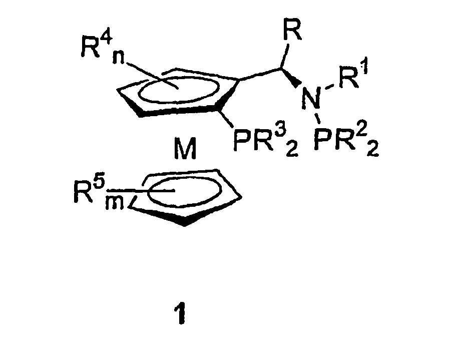 dans laquelle
R est choisi parmi un alkyle en C₁ à C₂₀ substitué et non substitué, ramifié et à chaîne linéaire, un cycloalkyle en C₃ à C₈ substitué et non substitué, un aryle carbocyclique en C₆ à C₂₀ substitué et non substitué, et un hétéroaryle en C₄ à C₂₀ substitué et non substitué dans lequel les hétéroatomes sont choisis parmi le soufre, l'azote, et l'oxygène ;
R¹, R², R³, R⁴, et R⁵ sont indépendamment choisis parmi l'hydrogène, un alkyle en C₁ à C₂₀ substitué et non substitué, ramifié et à chaîne linéaire, un cycloalkyle en C₃ à C₈ substitué et non substitué, un aryle carbocyclique en C₆ à C₂₀ substitué et non substitué, et un hétéroaryle en C₄ à C₂₀ substitué et non substitué dans lequel les hétéroatomes sont choisis parmi le soufre, l'azote, et l'oxygène ;
n va de 0 à 3 ;
m va de 0 à 5 ; et
M est choisi parmi les métaux des Groupes IVB, VB, VIB, VIIB et VIII.

11. Catalyseur supporté selon la revendication 1, dans lequel la phosphine - aminophosphine sensiblement énantiomériquement pure a la formule 2 : 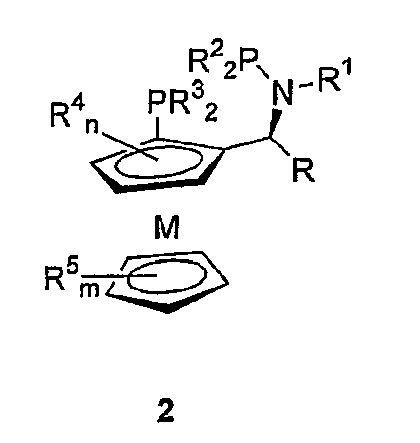 dans laquelle
R est choisi parmi un alkyle en C₁ à C₂₀ substitué et non substitué, ramifié et à chaîne linéaire, un cycloalkyle en C₃ à C₈ substitué et non substitué, un aryle carbocyclique en C₆ à C₂₀ substitué et non substitué, et un hétéroaryle en C₄ à C₂₀ substitué et non substitué dans lequel les hétéroatomes sont choisis parmi le soufre, l'azote, et l'oxygène ;
R¹, R², R³, R⁴, et R⁵ sont indépendamment choisis parmi l'hydrogène, un alkyle en C₁ à C₂₀ substitué et non substitué, ramifié et à chaîne linéaire, un cycloalkyle en C₃ à C₈ substitué et non substitué, un aryle carbocyclique en C₆ à C₂₀ substitué et non substitué, et un hétéroaryle en C₄ à C₂₀ substitué et non substitué dans lequel les hétéroatomes sont choisis parmi le soufre, l'azote, et l'oxygène ;
n va de 0 à 3;
m va de 0 à 5; et
M est choisi parmi les métaux des Groupes IVB, VB, VIB, VIIB et VIII.

12. Procédé pour la préparation du catalyseur supporté selon la revendication 1, comprenant les étapes consistant :
(a) à mettre en contact un support avec un agent d'ancrage de type hétéropolyacide ou hétéropolyanion dans des conditions efficaces pour former un support contenant un hétéropolyacide ou un hétéropolyanion ; et
(b) à mettre en contact un complexe métallique d'une phosphine - aminophosphine sensiblement énantiomériquement pure avec le support contenant un hétéropolyacide ou un hétéropolyanion dans des conditions efficaces pour former un catalyseur supporté.

13. Procédé selon la revendication 12, dans lequel l'étape (a) se déroule dans un solvant à une température allant d'environ -25 °C à environ 250 °C pendant une période de temps allant d'environ 1 minute à environ 50 heures.

14. Procédé selon la revendication 12, dans lequel l'étape (b) se déroule dans un solvant à une température allant d'environ -25 °C à environ 250 °C pendant une période de temps allant d'environ 1 minute à environ 50 heures.

15. Procédé selon la revendication 13, dans lequel le complexe métallique est mis en contact avec le support contenant un hétéropolyacide ou un hétéropolyanion à une concentration destinée à fournir un rapport molaire du complexe métallique sur l'hétéropolyacide ou l'hétéropolyanion allant d'environ 0,1 : 1 à environ 6 : 1.

16. Procédé pour la préparation du catalyseur supporté selon la revendication 1, comprenant les étapes consistant :
(a) à mettre en contact un hétéropolyacide ou un hétéropolyanion avec un complexe métallique d'une phosphine - aminophosphine sensiblement énantiomériquement pure dans des conditions efficaces pour former un mélange contenant l'hétéropolyacide ou l'hétéropolyanion et le complexe métallique ; et
(b) à mettre en contact un support avec le mélange formé dans l'étape (a) dans des conditions efficaces pour former un catalyseur supporté.

17. Procédé selon la revendication 16, dans lequel l'étape (a) se déroule dans un solvant à une température allant d'environ -25 °C à environ 250 °C pendant une période de temps allant d'environ 1 minute à environ 50 heures.

18. Procédé selon la revendication 16, dans lequel le complexe métallique est mis en contact avec l'hétéropolyacide ou l'hétéropolyanion à une concentration destinée à fournir un rapport molaire du complexe métallique sur l'hétéropolyacide ou l'hétéropolyanion allant d'environ 0,1 : 1 à environ 6 : 1.

19. Procédé selon la revendication 16, dans lequel l'étape (b) se déroule dans un solvant à une température allant d'environ -25 °C à environ 250 °C pendant une période de temps allant d'environ 1 minute à environ 50 heures.

20. Procédé selon la revendication 16, dans lequel l'hétéropolyacide ou l'hétéropolyanion et le complexe métallique sont présents dans un rapport pondéral allant d'environ 0,1 : 1 à environ 20 : 1 par rapport au support utilisé dans l'étape (b).

21. Procédé pour la préparation du catalyseur supporté selon la revendication 1, comprenant les étapes consistant :
(a) à mettre en contact un support avec un hétéropolyacide ou un hétéropolyanion dans des conditions efficaces pour former un support contenant un hétéropolyacide ou un hétéropolyanion ;
(b) à mettre en contact ledit support contenant un hétéropolyacide ou un hétéropolyanion avec une matière de type précurseur de catalyseur dans des conditions efficaces pour former un précurseur de catalyseur supporté ; et
(c) à mettre en contact ledit précurseur de catalyseur supporté avec un ligand de type phosphine - aminophosphine sensiblement énantiomériquement pure pour fournir un catalyseur supporté.

22. Procédé selon la revendication 21, dans lequel le support utilisé dans l'étape (a) est calciné ou traité avec un alcoxyde métallique avant utilisation.

23. Procédé selon la revendication 22, dans lequel l'alcoxyde métallique est choisi dans le groupe comprenant l'alcoxyde de titane, l'alcoxyde d'aluminium, l'alcoxyde de silane, et l'alcoxyde de vanadium.

24. Procédé selon la revendication 21, dans lequel l'étape (a) se déroule dans un solvant à une température allant d'environ -25 °C à environ 250 °C pendant une période de temps allant d'environ 1 minute à environ 50 heures.

25. Procédé selon la revendication 21, dans lequel ladite matière de type précurseur de catalyseur est un sel ou un complexe métallique dont une entité catalytiquement active peut être préparée.

26. Procédé selon la revendication 25, dans lequel le sel ou le complexe métallique dudit précurseur de catalyseur est choisi dans le groupe comprenant le dimère de rhodium et de cyclooctadiène, le tétrafluoroborate de bis (cyclooctadiène) rhodium, le trifluorométhane sulfonate de bis (cyclooctadiène) rhodium, le tétrafluoroborate de bis (norbornadiène) rhodium, le dimère de ruthénium et de cyclooctadiène, le dimère de chlorure d'allylpalladium, et le chlorure de rhodium.

27. Procédé selon la revendication 21, dans lequel l'étape (b) se déroule dans un solvant à une température allant d'environ -25 °C à environ 250 °C pendant une période de temps allant d'environ 1 minute à environ 50 heures.

28. Procédé selon la revendication 21, dans lequel l'étape (c) se déroule dans un solvant à une température allant d'environ -25 °C à environ 250 °C pendant une période de temps allant d'environ 1 minute à environ 50 heures.

29. Procédé selon la revendication 21, dans lequel une quantité allant d'environ 1 mmole à environ 6 mmoles de ligand par mmole de matière de type précurseur de catalyseur supporté est utilisée dans l'étape (c).

30. Procédé pour l'hydrogénation d'un acide ou d'un ester α,β-insaturé, qui comprend la mise en contact d'un acide ou d'un ester α,β-insaturé prochiral avec le catalyseur supporté selon la revendication 1 et de l'hydrogène dans des conditions de température et de pression d'hydrogénation.
